# FASCICULE DE BREVET EUROPEEN

(11) **EP 2 816 974 B1**
(45) Date de publication et mention de la délivrance du brevet: **26.04.2017**
(21) Numéro de dépôt: 13710481.6
(22) Date de dépôt: 19.02.2013
(51) Int. Cl.: A61F 2/30, A61F 2/34, A61L 27/56

(54) **IMPLANT ORTHOPEDIQUE ET PROCEDE POUR FABRIQUER UN TEL IMPLANT ORTHOPEDIQUE**
ORTHOPÄDISCHES IMPLANTAT UND VERFAHREN ZUR HERSTELLUNG EINES SOLCHEN ORTHOPÄDISCHEN IMPLANTATS
ORTHOPAEDIC IMPLANT AND METHOD FOR PRODUCING SUCH AN ORTHOPAEDIC IMPLANT

(30) Priorité: 20.02.2012 FR 1251516
(43) Date de publication de la demande: 31.12.2014
(73) Titulaire: Brax, Michel, 67670 Mommenheim (FR); Charissoux, Jean-Louis, 87000 Limoges (FR); Lustig, Sébastien, 69004 Lyon (FR); Maman, Pascal, 13006 Marseille (FR); Roche, Olivier, 54710 Ludres (FR); Venet, Guillaume, 85140 St Martin Des Noyers (FR)
(72) Inventeur: Brax, Michel, 67670 Mommenheim (FR); Charissoux, Jean-Louis, 87000 Limoges (FR); Lustig, Sébastien, 69004 Lyon (FR); Maman, Pascal, 13006 Marseille (FR); Roche, Olivier, 54710 Ludres (FR); Venet, Guillaume, 85140 St Martin Des Noyers (FR)
(74) Mandataire: Chevalier, Renaud Philippe
(86) Numéro de dépôt international: PCT/FR2013/050336
(87) Numéro de publication internationale: WO 2013/124577

(56) Documents cités:
- EP-A1- 1 647 242
- WO-A1-02/17820
- US-A- 4 542 539
- US-A- 5 024 670

## Description

La présente invention concerne un implant orthopédique, tel qu'un cotyle pour prothèse de hanche. Par ailleurs, la présente invention concerne un procédé, pour fabriquer un implant orthopédique, tel qu'un cotyle pour une prothèse de hanche.

La présente invention trouve notamment application dans le domaine de la chirurgie réparatrice et de l'orthopédie, en particulier pour la fabrication de prothèses de hanche.

Les caractéristiques du préambule de la revendication 1 sont connues du document WO 02/17820 A1.

US5024670 décrit un implant orthopédique et son procédé de fabrication. L'implant comporte un substrat polymère présentant une surface externe destinée à être solidarisée à un tissu osseux. La surface externe est couverte par des particules de titane de taille relativement grande, car leur granulométrie est comprise entre 177 µm et 250 µm.

Cependant, comme cette grande taille des particules de titane induit une distribution des particules qui n'est pas uniforme sur la surface externe. En effet, le dépôt de ces grandes particules génère des interstices de dimensions très variables. Par conséquent, la croissance osseuse inefficace sur une telle surface externe réalise un mauvais accrochage de l'os, ce qui réduit la durée de service de la prothèse.

La présente invention vise notamment à résoudre, en tout ou partie, les problèmes mentionnés ci-avant.

A cet effet, l'invention a pour objet un implant orthopédique, tel qu'un cotyle pour prothèse de hanche, l'implant orthopédique comportant au moins un substrat qui comprend au moins un matériau plastique polymère et qui présente une surface externe destinée à être solidarisée à un tissu osseux, ladite surface externe étant partiellement couverte par des particules d'au moins un matériau métallique comprenant du titane ;

l'implant orthopédique étant caractérisé en ce que lesdites particules comprennent des particules primaires et des particules secondaires, les particules primaires présentant une granulométrie s'étendant entre 180 µm et 600 µm, de préférence entre 200 µm et 500 µm, les particules secondaires présentant une granulométrie s'étendant entre 70 µm et 145 µm, de préférence entre 90 µm et 125 µm, les particules primaires et les particules secondaires étant distribuées de manière relativement uniforme sur la surface externe, les particules secondaires étant logées dans des interstices se trouvant entre les particules primaires. En d'autres termes, la surface externe est couverte de petites particules et de grandes particules.

Ainsi, un tel recouvrement permet d'augmenter, voire de maximiser, le taux de recouvrement et la quantité d'interstices, donc de favoriser la croissance osseuse et l'accrochage du tissu osseux, ce qui conduit à une longue durée de service de la prothèse.

Dans la présente demande, le terme « granulométrie » fait référence à la taille des particules ou « grains ». La granulométrie est caractérisée de manière usuelle par un spectre granulométrique caractéristique d'une distribution numérique des particules d'un ensemble en fonction de la dimension de chaque particule.

Selon une variante de l'invention, la surface externe peut être partiellement couverte par d'autres particules, métalliques ou non, et présentant une granulométrie distincte, par exemple intermédiaire entre celles des particules primaires et secondaires.

Ainsi, ces autres particules permettent de s'adapter à des zones ayant des densités osseuses différentes.

Selon un mode de réalisation de l'invention, ledit matériau plastique polymère est sélectionné dans le groupe constitué d'un polyéthylène (PE), d'un d'un polyéthylène à très haut poids moléculaire (UHMW-PE), d'un polyéthylène hautement réticulé (XLPE), d'un polyéthylène vitaminé E, d'un polyuréthane et d'un polyétheréthercétone (PEEK).

Ainsi, un tel matériau plastique polymère permet de réaliser un substrat biocompatible, léger et résistant mécaniquement et chimiquement.

Selon un mode de réalisation de l'invention, le ou chaque matériau métallique est sélectionné dans le groupe constitué du titane pur, d'un alliage de titane de chrome, de cobalt et d'acier inoxydable tel que l'acier 316LVM.

Ainsi, un tel matériau métallique permet de favoriser l'accrochage des tissus osseux croissant autour de la surface externe.

Selon un mode de réalisation de l'invention, les particules primaires et les particules secondaires sont composées du même matériau métallique.

Ainsi, de telles particules primaires et particules secondaires peuvent être mises en oeuvre de la même manière, par exemple à la même température et/ou à la même pression.

Selon un mode de réalisation de l'invention, la superficie de la partie de la surface externe qui n'est pas couverte par lesdites particules représente entre 15% et 30%, de préférence entre 20% et 25%, de la superficie totale de la surface externe.

En d'autres termes, le taux de couverture de la surface externe par le matériau métallique est compris entre 60% et 80%, de préférence entre 65% et 75%. Ainsi, un tel taux de couverture permet l'accrochage et la croissance de nombreux tissus osseux, ce qui n'est pas le cas lorsque les particules métalliques sont trop petites et induisent un taux de couverture trop élevé.

Selon une variante de l'invention, les interstices primaires entre particules primaires ont des superficies approximativement égales.

Ainsi, de tels interstices permettent l'implantation des particules secondaires, plus petites, ce qui garantit des distributions uniformes des particules primaires et secondaires sur la surface externe. Dans la présente demande, le terme « approximativement égales » indique par exemple que le les superficies des interstices primaires varient de plus ou moins 20% par rapport à leur médiane.

Selon un mode de réalisation de l'invention, le nombre de particules primaires représente sensiblement entre 5% et 50%, de préférence entre 10% et 30%, de la somme du nombre de particules primaires et du nombre de particules secondaires.

La somme du nombre de particules primaires et du nombre de particules secondaires correspond globalement au nombre total de particules.

Ainsi, de telles proportions de particules primaires et secondaires permettent de réaliser une croissance osseuse dense et uniforme.

Corollairement, le nombre de particules secondaires représente sensiblement entre 95% et 50%, de préférence entre 70% et 30%, de la somme du nombre de particules primaires et du nombre de particules secondaires.

Selon un mode de réalisation de l'invention, la surface externe a globalement la forme d'une portion sphéroïdale, de préférence la forme d'une demi-sphère.

Ainsi, une surface externe hémisphérique permet de réaliser un accrochage isotrope, c'est-à-dire un accrochage résistant à des efforts exercés suivant différentes directions.

Selon un mode de réalisation de l'invention, l'implant orthopédique comporte un seul substrat monobloc.

Ainsi, un tel substrat monobloc est relativement aisé à réaliser.

Par ailleurs, la présente invention a pour objet un procédé, pour fabriquer un implant orthopédique, tel qu'un cotyle pour une prothèse de hanche, l'implant orthopédique comprenant au moins un matériau plastique polymère présentant une surface externe destinée à être solidarisée à un tissu osseux, le procédé comprenant les étapes :
- chauffer ladite surface externe à une température de ramollissement du matériau plastique polymère, le substrat étant de préférence placé dans un moule ;
- couvrir partiellement ladite surface externe par des particules primaires d'au moins un matériau métallique comprenant du titane, les particules primaires présentant une granulométrie s'étendant entre 180 µm et 600 µm, de préférence entre 200 µm et 500 µm, les particules primaires étant distribuées de manière relativement uniforme sur la surface externe ;
- presser une matrice chauffée contre la surface externe, de façon à solidariser les particules primaires à la surface externe ;
- couvrir partiellement ladite surface externe par des particules secondaires d'au moins un matériau métallique comprenant du titane, les particules secondaires présentant une granulométrie s'étendant entre 70 µm et 145 µm, de préférence entre 90 µm et 125 µm, les particules secondaires étant distribuées de manière relativement uniforme sur la surface externe ; et
- presser la matrice chauffée contre la surface externe, de façon à solidariser les particules secondaires à la surface externe.

Ainsi, un tel procédé permet de fabriquer un implant orthopédique conforme à l'invention de manière fiable et rapide. En particulier, un tel procédé ne met pas en oeuvre de dépôt par plasma, particulièrement énergivore et aléatoire.

Selon un mode de réalisation de l'invention, l'étape de couverture de la surface externe par les particules primaires est réalisée avant l'étape de couverture de la surface externe par les particules secondaires.

En d'autres termes, les particules secondaires (petites) complètent les emplacements laissés vacants par les particules primaires (grandes).

Ainsi, une telle succession de ces étapes permet de répartir de manière particulièrement uniforme les particules primaires et secondaires.

Selon une variante de l'invention, les particules primaires et les particules secondaires sont préalablement mélangées pour former une poudre homogène, et dans lequel l'étape de couverture de la surface externe par les particules primaires est réalisée simultanément à l'étape de couverture de la surface externe par les particules secondaires.

Ainsi, une telle simultanéité de ces étapes permet de fabriquer rapidement un implant orthopédique conforme à l'invention.

Selon un mode de réalisation de l'invention, l'étape de couverture de la surface externe par les particules primaires et l'étape de couverture de la surface externe par les particules secondaires sont réalisées par mise en contact dans un volume clos mis sous pression et chauffé à une température inférieure à la température de fusion du matériau plastique polymère.

Ainsi, un tel procédé de dépôt est rapide à effectuer.

Les modes de réalisation et les variantes mentionnés ci-avant peuvent être pris isolément ou selon toute combinaison techniquement admissible.

La présente invention sera bien comprise et ses avantages ressortiront aussi à la lumière de la description qui va suivre, donnée uniquement à titre d'exemple non limitatif et faite en référence aux dessins annexés, dans lesquels :
- la figure 1 est une vue en perspective d'un implant orthopédique conforme à l'invention ;
- la figure 2 est une vue en perspective, tronquée suivant le plan Il à la figure 1 et suivant un angle différent de la figure 1, de l'implant orthopédique de la figure 1 ;
- la figure 3 est une vue de dessus de l'implant orthopédique de la figure 1 ;
- la figure 4 est une photographie microscopique d'une partie de l'implant orthopédique de la figure 1 ;
- la figure 5 est une photographie microscopique, à la moitié de l'échelle de la figure 4, d'une partie de l'implant orthopédique de la figure 1 ; et
- la figure 6 est une vue schématique illustrant une étape d'un procédé conforme à l'invention, pour fabriquer l'implant orthopédique des figures 1 à 5.

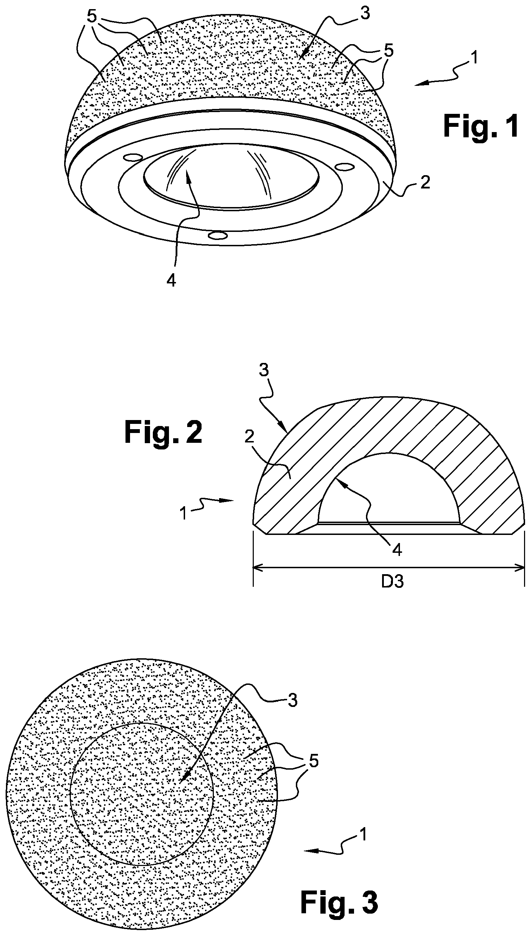

La figure 1 illustre un implant orthopédique 1, qui forme un cotyle pour prothèse de hanche. En d'autres termes, l'implant orthopédique 1 est un implant acétabulaire. L'implant orthopédique 1 comporte un substrat 2 comprenant au moins un matériau plastique polymère. Dans l'exemple des figures, l'implant orthopédique 1 comporte seulement le substrat 2, qui est monobloc.

Le matériau plastique polymère formant le substrat 2 est un polyéthylène à haute densité (PEHD). En pratique, ce matériau plastique polymère peut être sélectionné dans le groupe constitué d'un polyéthylène à haute densité (PEHD), d'un polyéthylène hautement réticulé (XLPE), d'un polyuréthane et d'un polyétheréthercétone (PEEK).

Le substrat 2 présente une surface externe 3 qui est destinée à être solidarisée à un tissu osseux appartenant à l'os iliaque non représenté. Comme le montre la figure 2, la surface externe 3 a globalement la forme d'un demi-sphèroïde voire d'une demi-sphère, destinée à être incorporée à l'os iliaque. La surface externe 3 a un diamètre D3 d'environ 50 mm. Alternativement, le diamètre de la surface externe peut être sélectionné entre 40 mm et 70 mm.

Le substrat 2 présente en outre une surface interne 4 située à l'opposé de la surface externe 3. La surface interne 4 forme une surface cotyloïdienne, pour l'articulation d'une tête fémorale non représentée.

Comme le montre la figure 3, la surface externe 3 est en partie couverte par des particules 5 d'un matériau métallique composées ici de titane pur. En pratique, le ou chaque matériau métallique peut être un alliage de titane, donc un matériau comprenant du titane et un autre matériau métallique ou non, par exemple de chrome, de cobalt et d'acier inoxydable tel que l'acier 316LVM. Par exemple, de tels alliages sont définis dans la norme ISO 5832.

Une partie de la surface externe 3 n'est pas couverte par les particules 5. La superficie de cette partie de la surface externe 3 non couverte par les particules 5 représente environ 25% de la superficie totale de la surface externe 3. En d'autres termes, les particules 5 couvrent environ 75% de la superficie totale de la surface externe 3. Dans l'exemple des figures, la superficie totale de la surface externe 3 est d'environ 3930 mm², tandis que la superficie de cette partie de la surface externe 3 non couverte.

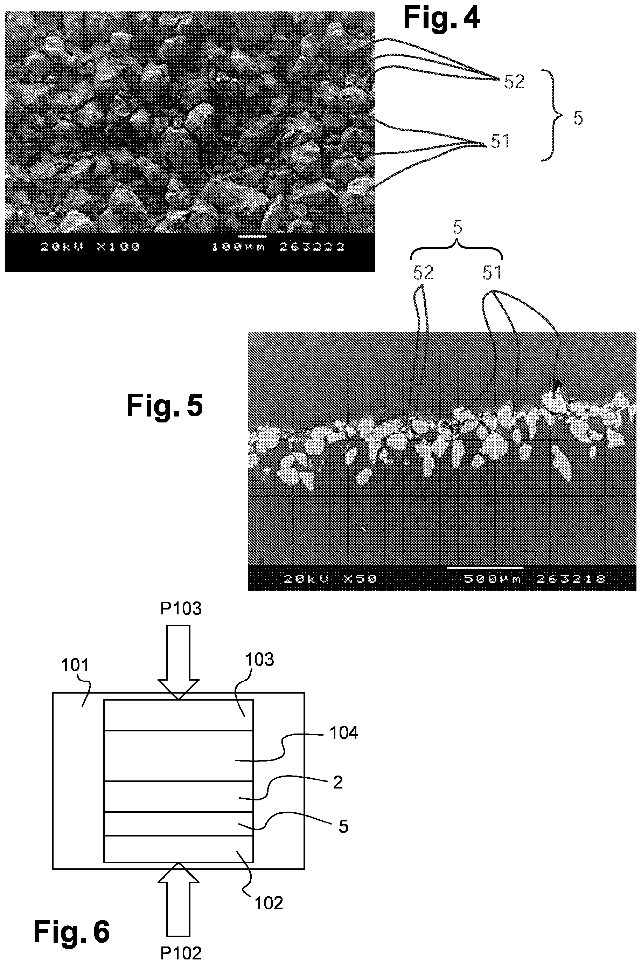

Comme le montrent les figures 4 et 5, les particules 5 comprennent principalement des particules primaires 51 et des particules secondaires 52. Dans l'exemple des figures, les particules primaires 51 et les particules secondaires 52 sont composées du même matériau métallique, du titane pur.

Le nombre de particules primaires 51 représente sensiblement entre 5% et 50%, de préférence entre 10% et 30%, de la somme du nombre de particules primaires 51 et du nombre de particules secondaires 52, c'est-à-dire environ au nombre total de particules 5. Corollairement, le nombre de particules secondaires 52 représente sensiblement entre 95% et 50%, de préférence entre 70% et 30%, de la somme du nombre de particules primaires 51 et du nombre de particules secondaires 52.

Dans l'exemple des figures, la masse totale de particules primaires 51 et secondaires 52 peut être par exemple comprise entre 4 g et 20 g.

Les particules primaires 51 présentent une granulométrie s'étendant substantiellement entre 200 µm et 500 µm. Les particules secondaires 52 présentent une granulométrie s'étendant substantiellement entre 90 µm et 125 µm.

En pratique, les particules primaires 51 présentent une granulométrie s'étendant substantiellement entre 180 µm et 600 µm. En pratique, les particules secondaires 52 présentent une granulométrie s'étendant substantiellement entre 70 µm et 145 µm.

Les particules primaires 51 et les particules secondaires 52 sont distribuées de manière relativement uniforme sur la surface externe 3. Ainsi, les interstices primaires entre particules primaires 51 ont des superficies approximativement égales. Ces interstices primaires logent les particules secondaires 52.

Comme l'illustre la figure 6, un procédé conforme à l'invention, pour fabriquer l'implant orthopédique 1, comprend les étapes :
- placer le substrat 2 dans un moule 101, ce qui permet notamment de réaliser le procédé de fabrication sous atmosphère contrôlée ;
- chauffer la surface externe 3 à une température de ramollissement du polyéthylène haute densité (PEHD) ;
- couvrir partiellement la surface externe 3 par des particules primaires 51, de sorte que les particules primaires 51 sont distribuées de manière relativement uniforme sur la surface externe 3 ;
- presser une matrice 102 chauffée contre la surface externe 3, de façon à solidariser les particules primaires 51 à la surface externe.

En outre, comme le montre la figure 6, un tel procédé de fabrication comprend les étapes :
- couvrir partiellement la surface externe 3 par des particules secondaires 51, de sorte que les particules secondaires 52 sont distribuées de manière relativement uniforme sur la surface externe 3 ; et
- presser la matrice 102 chauffée contre la surface externe 3, de façon à solidariser les particules secondaires 52 à la surface externe 3.

Dans le procédé conforme à l'invention, l'étape de couverture de la surface externe 3 par les particules primaires 51 est réalisée avant l'étape de couverture de la surface externe 3 par les particules secondaires 52.

Alternativement, les particules primaires et les particules secondaires peuvent être préalablement mélangées pour former une poudre homogène. Ainsi, l'étape de couverture de la surface externe par les particules primaires peut être réalisée simultanément à l'étape de couverture de la surface externe par les particules secondaires.

L'étape de couverture de la surface externe par les particules primaires et l'étape de couverture de la surface externe par les particules secondaires sont réalisées par mise en contact dans le volume clos du moule 101 qui est mis sous pression et chauffé à une température inférieure à la température de fusion du matériau plastique polymère. À cet effet, des matrices 102 et 103 peuvent exercer des pressions P102 et P103 sur le moule 101. Une entretoise 104 distribue les pressions P102 et P103.

En service, l'implant orthopédique 1 forme un cotyle pour prothèse de hanche ou implant acétabulaire. L'implant orthopédique 1 est solidarisé à un tissu osseux appartenant à l'os iliaque non représenté. L'articulation de la tête fémorale se fait sur la surface interne 4.

## Revendications

1. Implant orthopédique (1), tel qu'un cotyle pour prothèse de hanche, l'implant orthopédique (1) comportant au moins un substrat (2) qui comprend au moins un matériau plastique polymère et qui présente une surface externe (3) destinée à être solidarisée à un tissu osseux, ladite surface externe (3) étant partiellement couverte par des particules (5) d'au moins un matériau métallique comprenant du titane ;
l'implant orthopédique (1) étant **caractérisé en ce que** lesdites particules (5) comprennent des particules primaires (51) et des particules secondaires (52), les particules primaires (51) présentant une granulométrie s'étendant entre 180 µm et 600 µm, de préférence entre 200 µm et 500 µm, les particules secondaires (52) présentant une granulométrie s'étendant entre 70 µm et 145 µm, de préférence entre 90 µm et 125 µm, les particules primaires (51) et les particules secondaires (52) étant distribuées de manière relativement uniforme sur la surface externe (3), les particules secondaires (52) étant logées dans des interstices se trouvant entre les particules primaires (51).

2. Implant orthopédique (1) selon la revendication 1, dans lequel ledit matériau plastique polymère est sélectionné dans le groupe constitué d'un polyéthylène (PE), d'un polyéthylène à très haut poids moléculaire (UHMW-PE), d'un polyéthylène hautement réticulé (XLPE), d'un polyéthylène vitaminé E, d'un polyuréthane et d'un polyétheréthercétone (PEEK).

3. Implant orthopédique (1) selon l'une des revendications précédentes, dans lequel le ou chaque matériau métallique est sélectionné dans le groupe constitué du titane pur, d'un alliage de titane, de chrome, de cobalt et d'acier inoxydable tel que l'acier 316LVM.

4. Implant orthopédique (1) selon l'une des revendications précédentes, dans lequel les particules primaires (51) et les particules secondaires (52) sont composées du même matériau métallique.

5. Implant orthopédique (1) selon l'une des revendications précédentes, dans lequel la superficie de la partie de la surface externe (3) qui n'est pas couverte par lesdites particules (5) représente entre 15% et 30%, de préférence entre 20% et 25%, de la superficie totale de la surface externe (3).

6. Implant orthopédique (1) selon l'une des revendications précédentes, dans lequel le nombre de particules primaires (51) représente sensiblement entre 5% et 50%, de préférence entre 10% et 30%, de la somme du nombre de particules primaires (51) et du nombre de particules secondaires (52).

7. Implant orthopédique (1) selon l'une des revendications précédentes, dans lequel la surface externe (3) a globalement la forme d'une portion sphéroïdale, de préférence la forme d'une demi-sphère.

8. Implant orthopédique (1) selon l'une des revendications précédentes, comportant un seul substrat (2) monobloc.

9. Procédé, pour fabriquer un implant orthopédique (1) selon la revendication 1, tel qu'un cotyle pour une prothèse de hanche, l'implant orthopédique (1) comprenant au moins un matériau plastique polymère présentant une surface externe (3) destinée à être solidarisée à un tissu osseux, le procédé comprenant les étapes :
- chauffer ladite surface externe (3) à une température de ramollissement du matériau plastique polymère, le substrat (2) étant de préférence placé dans un moule (101) ;
- couvrir partiellement ladite surface externe (3) par des particules primaires (51) d'au moins un matériau métallique comprenant du titane, les particules primaires (51) présentant une granulométrie s'étendant entre 180 µm et 600 µm, de préférence entre 200 µm et 500 µm, les particules primaires (51) étant distribuées de manière relativement uniforme sur la surface externe (3) ;
- presser une matrice (102) chauffée contre la surface externe (3), de façon à solidariser les particules primaires (51) à la surface externe (3) ;
- couvrir partiellement ladite surface externe (3) par des particules secondaires (52) d'au moins un matériau métallique comprenant du titane, les particules secondaires (52) présentant une granulométrie s'étendant entre 70 µm et 145 µm, de préférence entre 90 µm et 125 µm, les particules secondaires (52) étant distribuées de manière relativement uniforme sur la surface externe (3) ; et
- presser la matrice (102) chauffée contre la surface externe (3), de façon à solidariser les particules secondaires (52) à la surface externe (3).

10. Procédé selon la revendication 9, dans lequel l'étape de couverture de la surface externe (3) par les particules primaires (51) est réalisée avant l'étape de couverture de la surface externe (3) par les particules secondaires (52).

11. Procédé selon l'une des revendications 9 à 10, dans lequel l'étape de couverture de la surface externe (3) par les particules primaires (51) et l'étape de couverture de la surface externe (3) par les particules secondaires (52) sont réalisées par mise en contact dans un volume clos mis sous pression et chauffé à une température inférieure à la température de fusion du matériau plastique polymère.

## Patentansprüche

1. Orthopädisches Implantat (1), wie zum Beispiel eine Gelenkpfanne für Hüftprothese, wobei das orthopädische Implantat (1) mindestens ein Substrat (2) umfasst, das mindestens ein Polymerkunststoffmaterial umfasst, das eine äußere Oberfläche (3) aufweist, die dazu bestimmt ist, fest mit einem Knochengewebe verbunden zu sein, wobei die äußere Oberfläche (3) teilweise mit Partikeln (5) aus mindestens einem metallischen Material, das Titan enthält, bedeckt ist,
wobei das orthopädisches Implantat (1) **dadurch gekennzeichnet ist, dass** die Partikel (5) Primärpartikel (51) und Sekundärpartikel (52) umfassen, wobei die Primärpartikel (51) eine Granulometrie umfassen, die sich zwischen 180 µm und 600 µm, bevorzugt zwischen 200 µm und 500 µm erstreckt, wobei die Sekundärpartikel (52) eine Granulometrie aufweisen, die sich zwischen 70 µm und 145 µm erstreckt, bevorzugt zwischen 90 µm und 125 µm, wobei die Primärpartikel (51) und die Sekundärpartikel (52) relativ gleichförmig auf der äußeren Oberfläche (3) verteilt sind, wobei die Sekundärpartikel (52) in Zwischenräumen aufgenommen sind, die sich zwischen den Primärpartikeln (51) befinden.

2. Orthopädisches Implantat (1) nach Anspruch 1, wobei das Polymerkunststoffmaterial aus der Gruppe ausgewählt ist, die aus einem Polyethylen (PE), einem Polyethylen mit sehr hohem Molekulargewicht (UHMW-PE), einem hochvernetzten Polyethylen (XLPE), einem Polyethylen mit Vitamin E, einem Polyurethan und einem Polyetheretherketon (PEEK) besteht.

3. Orthopädisches Implantat (1) nach einem der vorhergehenden Ansprüche, wobei das oder jedes metallische Material aus der Gruppe ausgewählt ist, die aus reinem Titan, einer Titanlegierung, aus Chrom, Kobalt und rostfreiem Stahl, wie zum Beispiel dem Stahl 316 LVM, besteht.

4. Orthopädisches Implantat (1) nach einem der der vorhergehenden Ansprüche, wobei die Primärpartikel (51) und die Sekundärpartikel (52) aus demselben metallischen Material bestehen.

5. Orthopädisches Implantat (1) nach einem der vorhergehenden Ansprüche, wobei die Oberfläche des Teils der äußeren Oberfläche (3), der nicht von den Partikeln (5) bedeckt ist, zwischen 15 % und 30 %, bevorzugt zwischen 20 % und 25 % der Gesamtoberfläche der äußeren Oberfläche (3) darstellt.

6. Orthopädisches Implantat (1) nach einem der vorhergehenden Ansprüche, wobei die Anzahl von Primärpartikeln (51) im Wesentlichen zwischen 5 % und 50 %, bevorzugt zwischen 10 % und 30 % der Summe der Anzahl Primärpartikel (51) und der Anzahl Sekundärpartikel (52) darstellt.

7. Orthopädisches Implantat (1) nach einem der vorhergehenden Ansprüche, wobei die äußere Oberfläche (3) im Wesentlichen die Form eines kugelförmigen Anteils, bevorzugt die Form einer Halbkugel hat.

8. Orthopädisches Implantat (1) nach einem der vorhergehenden Ansprüche, das ein einziges einteiliges Substrat (2) umfasst.

9. Verfahren zum Herstellen eines orthopädischen Implantats (1) nach Anspruch 1, wie zum Beispiel einer Gelenkpfanne für eine Hüftprothese, wobei das orthopädische Implantat (1) mindestens ein Polymerkunststoffmaterial umfasst, das eine äußeren Oberfläche (3) aufweist, die dazu bestimmt ist, fest mit einem Knochengewebe verbunden zu sein, wobei das Verfahren die folgenden Schritte umfasst:
- Erhitzen der äußeren Oberfläche (3) auf eine Erweichungstemperatur des Polymerkunststoffmaterials, wobei das Substrat (2) bevorzugt in eine Form (101) platziert wird;
- teilweises Bedecken der äußeren Oberfläche (3) durch Primärpartikel (51) aus mindestens einem metallischen Material, das Titan umfasst, wobei die Primärpartikel (51) eine Granulometrie aufweisen, die sich zwischen 180 µm und 600 µm, bevorzugt zwischen 200 µm und 500 µm erstreckt, wobei die Primärpartikel (51) relativ gleichförmig auf der äußeren Oberfläche (3) verteilt sind;
- Pressen eines erhitzten Gesenks (102) gegen die äußere Oberfläche (3) derart, dass die Primärpartikel (51) mit der externen Oberfläche (3) fest verbunden werden;
- teilweises Bedecken der äußeren Oberfläche (3) mit Sekundärpartikeln (52) aus mindestens einem metallischen Material, das Titan umfasst, wobei die Sekundärpartikel (52) eine Granulometrie aufweisen, die sich zwischen 70 µm und 145 µm, bevorzugt zwischen 90 µm und 125 µm erstreckt, wobei die Sekundärpartikel (52) relativ gleichmäßig auf der äußeren Oberfläche (3) verteilt sind; und
- Drücken des erhitzten Gesenks (102) gegen die äußere Oberfläche (3) derart, dass die Sekundärpartikel (52) fest mit der äußeren Oberfläche (3) verbunden werden.

10. Verfahren nach Anspruch 9, wobei der Schritt des Bedeckens der äußeren Oberfläche (3) durch die Primärpartikel (51) vor dem Schritt des Bedeckens der äußeren Oberfläche (3) durch die Sekundärpartikel (52) ausgeführt wird.

11. Verfahren nach einem der Ansprüche 9 bis 10, wobei der Schritt des Bedeckens der äußeren Oberfläche (3) durch die Primärpartikel (51) und der Schritt des Bedeckens der externen Oberfläche (3) durch die Sekundärpartikel (52) durch Inberührungbringen in einem geschlossenen Raum, der mit Druck beaufschlagt und auf eine Temperatur unter der Fusionstemperatur des Polymerkunststoffmaterials erhitzt ist, ausgeführt wird.

## Claims

1. An orthopedic implant (1), such as an acetabular cup for a hip prosthesis, the orthopedic implant (1) including at least one substrate (2) which comprises at least one polymer plastic material and which has an outer surface (3) intended to be secured to a bone tissue, said outer surface (3) being partially covered with particles (5) of at least one metallic material comprising titanium;
the orthopedic implant (1) being **characterized in that** said particles (5) comprise primary particles (51) and secondary particles (52), the primary particles (51) having a particle size ranging from 180 µm to 600 µm, preferably from 200 µm to 500 µm, the secondary particles (52) having a particle size ranging from 70 µm to 145 µm, preferably from 90 µm to 125 µm, the primary particles (51) and the secondary particles (52) being distributed in a relatively uniform manner over the outer surface (3), the secondary particles (52) being housed in interstices located between the primary particles (51).

2. The orthopedic implant (1) according to claim 1, wherein said polymer plastic material is selected from the group constituted of polyethylene (PE), a ultra-high molecular weight polyethylene (UHMW-PE), a highly cross-linked polyethylene (XLPE), an E-vitaminized polyethylene, a polyurethane and a polyether ether ketone (PEEK).

3. The orthopedic implant (1) according to any of the preceding claims, wherein the or each metallic material is selected from the group consisting of pure titanium, an alloy of titanium, chromium, cobalt and stainless steel such as 316L VM steel.

4. The orthopedic implant (1) according to any of the preceding claims, wherein the primary particles (51) and the secondary particles (52) are composed of the same metallic material.

5. The orthopedic implant (1) according to any of the preceding claims, wherein the surface area of the outer surface (3) portion that is not covered with said particles (5) represents between 15% and 30%, preferably between 20% and 25%, of the total surface area of the outer surface (3).

6. The orthopedic implant (1) according to any of the preceding claims, wherein the number of primary particles (51) represents substantially between 5% and 50%, preferably between 10% and 30%, of the sum of the number of primary particles (51) and the number of secondary particles (52).

7. The orthopedic implant (1) according to any of the preceding claims, wherein the outer surface (3) has generally the shape of a spheroidal portion, preferably the shape of a half-sphere.

8. The orthopedic implant (1) according to any of the preceding claims, including one-piece single substrate (2).

9. A method for manufacturing an orthopedic implant (1) according to claim 1, such as an acetabular cup for a hip prosthesis, the orthopedic implant (1) comprising at least one polymer plastic material having an outer surface (3) intended to be secured to a bone tissue, the method comprising the steps consisting in:
- heating said outer surface (3) to a softening temperature of the polymer plastic material, the substrate (2) being preferably placed in a mold (101);
- partially covering said outer surface (3) with primary particles (51) of at least one metallic material comprising titanium, the primary particles (51) having a particle size ranging from 180 µm to 600 µm, preferably from 200 µm to 500 µm, the primary particles (51) being distributed in a relatively uniform manner over the outer surface (3);
- pressing a heated die (102) against the outer surface (3), so as to secure the primary particles (51) to the outer surface (3);
- partially covering said outer surface (3) with secondary particles (52) of at least one metallic material comprising titanium, the secondary particles (52) having a particle size ranging from 70 µm to 145 µm, preferably from 90 µm to 125 µm, the secondary particles (52) being distributed in a relatively uniform manner over the outer surface (3); and
- pressing the heated die (102) against the outer surface (3), so as to secure the secondary particles (52) to the outer surface (3).

10. The method according to claim 9, wherein the step of covering the outer surface (3) with the primary particles (51) is performed before the step of covering the outer surface (3) with the secondary particles (52).

11. The method according to any of claims 9 to 10, wherein the step of covering the outer surface (3) with the primary particles (51) and the step of covering the outer surface (3) with the secondary particles (52) are performed by putting in contact in an enclosed volume which is pressurized and heated at a temperature lower than the melting temperature of the polymer plastic material.
